# EUROPÄISCHE PATENTANMELDUNG

(11) **EP 1 046 632 A1**
(43) Veröffentlichungstag der Anmeldung: **25.10.2000**
(21) Anmeldenummer: 00107146.3
(22) Anmeldetag: 10.04.2000
(51) Int. Cl.: C07C 201/12

(54) **Verfahren zur Herstellung von 1-substituierten 2,4-Dinitrobenzolen**

(30) Priorität: 22.04.1999 DE 19918291
(71) Anmelder: Bayer Aktiengesellschaft, 51368 Leverkusen (DE)
(72) Erfinder: Rodefeld, Lars, Dr., 51371 Leverkusen (DE); Klausener, Alexander, Dr., 50259 Pulheim (DE); Hagedorn, Ferdinand, Dr., 51381 Leverkusen (DE)

(57) **Zusammenfassung**

Bereitgestellt wird ein Verfahren zur Herstellung von speziellen 1-substituierten 2,4-Dinitrobenzolen durch Umsetzung von 1-Halogen-2,4-dinitrobenzolen mit Mono-Alkalimetallsalzen von speziellen Diolen, wobei das 1-Halogen-2,4-dinitrobenzol und das Mono-Alkalimetallsalz des Diols simultan dosiert und zur Reaktion gebracht werden.

## Beschreibung

Die vorliegende Erfindung betrifft ein Verfahren zur Herstellung von 1-substituierten 2,4-Dinitrobenzolen durch Umsetzung von 1-Halogen-2,4-dinitrobenzolen mit Mono-Alkalimetallsalzen von Diolen.

1-substituierte 2,4-Dinitrobenzole finden in den unterschiedlichsten Gebieten Anwendung. Ihrer Synthese kommt daher besondere Bedeutung zu. 2-(2',4'-Dinitrophenoxy)-ethanol ist beispielsweise ein geeignetes Edukt zur Herstellung von 2-(2',4'-Diaminophenoxy)-ethanol und seinen Salzen, die in Oxidationsfärbemittel-zusammensetzungen als Meta-Komponente Verwendung finden (DE-OS-27 58 735 und DE-OS-27 37 138). 2-(2',4'-Dinitrophenoxy)-ethanol ist ferner auch ein exzellenter Weichmacher für Celluloseacetat.

Gemäß DE-OS-27 58 735 geht man zur Herstellung von 2-(2',4'-Dinitrophenoxy)-ethanol von einer Vorlage von Mono-Kalium-glykolat in Ethylenglykol aus, zu der 1-Chlor-2,4-dinitrobenzol zudosiert wird. Das Mono-Kalium-glykolat liegt dabei in einem 1,4-fachen molaren Überschuss vor. Mit diesem Verfahren wird 2-(2',4'-Dinitrophenoxy)-ethanol allerdings nur in einer Ausbeute von 68,6 % erhalten. Aus der Tatsache, dass der Schmelzpunkt des erhaltenen Produkts mit 100 bis 102°C angegeben wird, während reines, kristallines 2-(2',4'-Dinitrophenoxy)-ethanol gemäß DE-P-479 831 einen Schmelzpunkt von 111 bis 112°C besitzt, lässt sich schlussfolgern, dass das Produkt aus DE-OS-27 58 735 noch deutliche Verunreinigungen enthält.

Aus J. Chem. Soc. 1921 (119), 2076-8 ist ein weiteres Verfahren zur Herstellung von 2-(2',4'-Dinitrophenoxy)-ethanol bekannt, bei dem eine Lösung von 1-Chlor-2,4-dinitrobenzol in Ethylenglykol vorgelegt und in diese Vorlage eine Mischung aus Natriumhydroxid, Wasser und Ethylenglykol zudosiert wird. Die Mischung aus Natriumhydroxid, Wasser und Ethylenglykol wird dabei durch Lösen des festen Natriumhydroxids in Wasser und anschließende Zugabe des Ethylenglykols erhalten.

Mit diesem Verfahren wird eine Rohausbeute von 93 % erreicht, wobei das Rohprodukt zur Reinigung des 2-(2',4'-Dinitrophenoxy)-ethanols anschließend einer Umkristallisation aus Essigsäure unterworfen wird. Bei dieser Reaktion entsteht als unerwünschtes Nebenprodukt 1,2-Bis-(2',4'-dinitrophenoxy)-ethan. Es werden ferner Experimente beschrieben, bei denen die Mischung aus Natriumhydroxid, Wasser und Ethylenglykol nicht durch Zugabe des Ethylenglykols zur wässrigen Natriumhydroxid-Lösung, sondern umgekehrt durch Zugabe der wässrigen Natriumhydroxid-Lösung zum Ethylenglykol hergestellt wird. Es wird angegeben, dass dadurch die Entstehung des Nebenprodukts 1,2-Bis-(2',4'-dinitrophenoxy)-ethan vermieden werden kann. Eine Nacharbeitung dieser Versuche zeigte jedoch, dass auch bei dieser Variante 1,2-Bis-(2^{'},4'-dinitrophenoxy)-ethan mit 8 % der Gesamtausbeute entsteht.

In US-A-2,988,571 wird ein Verfahren zur Herstellung von 2-(2',4'-Dinitrophenoxy)-ethanol beschrieben, mit dem die Bildung der Nebenprodukte 1,2-Bis-(2',4'-dinitrophenoxy)-ethan und 1,2-Bis-(2',4'-dinitrophenoxy)-ethanol zurückgedrängt werden soll. Hierzu wird in eine Vorlage von Ethylenglykol bei Temperaturen von 30 bis 130°C, vorzugsweise 85 bis 90°C, gleichzeitig 1-Chlor-2,4-dinitrobenzol und festes, gepulvertes, weitgehend wasserfreies Natriumhydroxid jeweils in kleinen Portionen zudosiert. Wesentlich ist hierbei, dass neue Portionen der beiden Substanzen jeweils erst dann wieder hinzugefügt werden, wenn die zuvor zugegebenen abreagiert sind. Entscheidend ist weiterhin, dass das Molverhältnis des Ethylenglykols zum 1-Chlor-2,4-dinitrobenzol mindestens 3 beträgt und das molare Verhältnis des 1-Chlor-2,4-dinitrobenzols zum Natriumhydroxid bei ca. 1.1 bis 1.3 liegt. Anschließend wird dem Reaktionssystem Wasser in einer solchen Menge zugesetzt, dass das 2-(2',4'-Dinitrophenoxy)-ethanol als Niederschlag anfällt. In einer bevorzugten Ausführungsform des Verfahrens wird eine Vielzahl extrem kleiner Portionen zugegeben, so dass eine quasi kontinuierliche Zugabe des 1-Chlor-2,4-dinitrobenzols und des Natriumhydroxids resultiert. Auf diese Weise wird eine Ausbeute von 86,5 % 2-(2',4'-Dinitrophenoxy)ethanol bezogen auf das eingesetzte 1-Chlor-2,4-dinitrobenzol erhalten. Allerdings sind im Reaktionsprodukt trotzdem ca. 8 % des Nebenprodukts 1,2-Bis-(2',4'-dinitrophenoxy)-ethan enthalten (Test 1). Wird das molare Verhältnis des 1-Chlor-2,4-dinitrobenzols zum Natriumhydroxid über den angegebenen Wertebereich hinaus erhöht, so steigt der Anteil des Nebenprodukts 1,2-Bis-(2',4'-dinitrophenoxy)-ethan sogar auf 23 % (Test 2).

Insbesondere dann, wenn die 1-substituierten 2,4-Dinitrobenzole wie 2-(2',4'-Dinitrophenoxy)-ethanol im Kosmetikbereich eingesetzt werden, z.B. als Precursor-Verbindungen auf dem Gebiet der Haarkosmetik, ist die Reinheit der 1-substituierten 2,4-Dinitrobenzole ein entscheidender Faktor: Bereits geringe Kontaminationen durch Fremdstoffe sind aus medizinischen Gründen (z.B. Gefahr der Auslösung von Allergien) problematisch.

Die Aufgabe der vorliegenden Erfindung bestand somit darin, ein Verfahren bereitzustellen, welches die Herstellung von 1-substituierten 2,4-Dinitrobenzolen und insbesondere von 2-(2',4'-Dinitrophenoxy)-ethanol nicht nur mit sehr guten Ausbeuten, sondern auch mit hohen Reinheiten ermöglicht.

Diese Aufgabe wird gelöst durch ein Verfahren zur Herstellung von 1-substituierten 2,4-Dinitrobenzolen der Formel (I) 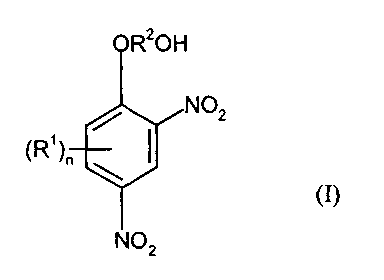
wobei R¹ unabhängig voneinander lineares oder verzweigtes C₁-C₂₀-Alkyl, C₆-C₁₈-Aryl, C₂-C₂₀-Acyl, COOH, COOR³, worin R³ für einen linearen oder verzweigten C₁-C₂₀-Alkylrest steht, SO₃H, SO₃R⁴, worin R⁴ für einen linearen oder verzweigten C₁-C₂₀-Alkylrest oder einen C₆-C₁₈-Arylrest, bei dem gegebenenfalls ein oder mehrere C-Atome durch O, S oder N ersetzt sind, steht, oder N(R⁵)₂, worin R⁵ unabhängig voneinander für Wasserstoff, einen linearen oder verzweigten C₁-C₂₀-Alkylrest oder einen C₂-C₂₀-Acylrest stehen, bedeuten, n eine ganze Zahl von 0 bis 3 ist und R² einen Rest der Formel (II) 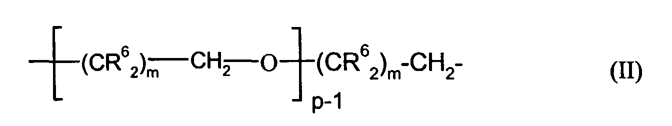
worin R⁶ unabhängig voneinander Wasserstoff oder einen C₁-C₅-Alkylrest bedeuten,
m eine ganze Zahl von 1 bis 12 und p eine ganze Zahl von 1 bis 4 darstellt,
oder einen Rest der Formel (III) bedeutet, 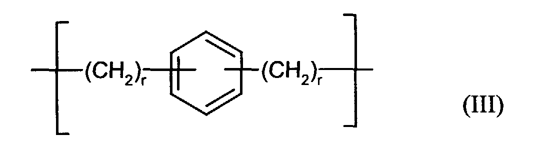
worin r unabhängig voneinander eine ganze Zahl von 0 bis 2 ist und gegebenenfalls ein oder mehrere Kohlenstoff-Atome des Phenylrings durch N, O oder S ersetzt sind,
durch Umsetzung von 1-Halogen-2,4-dinitrobenzolen der Formel (IV) 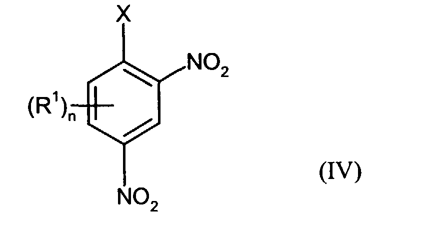
wobei X Halogen bedeutet und R¹ und n die für die Formel (I) genannten Bedeutungen besitzen,
mit Mono-Alkalimetallsalzen von Diolen der Formel (V)

   HO-R²-OH (V)
wobei R² die für die Formel (I) genannten Bedeutungen besitzt,
   welches dadurch gekennzeichnet ist, dass das 1-Halogen-2,4-dinitrobenzol der Formel (IV) und das Mono-Alkalimetallsalz des Diols der Formel (V) simultan dosiert und zur Reaktion gebracht werden.

Das erfindungsgemäße Verfahren mit seiner essentiellen simultanen Dosierweise für das 1-Halogen-2,4-dinitrobenzol der Formel (IV) und das Mono-Alkalimetallsalz des Diols der Formel (V) zeichnet sich dadurch aus, dass es die 1-substituierten 2,4-Dinitrobenzole der Formel (I) mit einer Reinheit von mindestens 90 %, bevorzugt mindestens 92 % und einer Ausbeute von mindestens 88 % liefert. Eine Umkristallisation, wie in J. Chem. Soc. 1921 (119), 2076-8 beschrieben, ist nicht mehr notwendig.

In den 1-Halogen-2,4-dinitrobenzolen der Formel (IV) steht X bevorzugt für Fluor, Chlor, Brom oder Iod, insbesondere für Chlor. R¹ bedeutet unabhängig voneinander bevorzugt lineares oder verzweigtes C₁-C₁₀-Alkyl, C₆-C₁₂-Aryl, C₂-C₆-Acyl oder N(R⁵)₂, worin R⁵ unabhängig voneinander für Wasserstoff, einen linearen oder verzweigten C₁-C₆-Alkylrest oder einen C₂-C₆-Acylrest stehen. Ferner ist n bevorzugt gleich 0, 1 oder 2.

Die im erfindungsgemäßen Verfahren eingesetzten 1-Halogen-2,4-dinitrobenzole der Formel (IV) sind zum Teil kommerziell erhältlich oder aber auf dem Fachmann bekannte Art und Weise herstellbar. Sie können als Schmelze, Feststoff oder als Lösung in einem organischen Lösungsmittel A zudosiert werden. Als organisches Lösungsmittel A sind Ether, gegebenenfalls substituierte aliphatische Kohlenwasserstoffe, wie Nitroalkane oder Methylenchlorid, gegebenenfalls substituierte aromatische Kohlenwasserstoffe, wie Toluol, Nitroaromaten oder Chloraromaten, Amide und Ethylenglykol geeignet. Als besonders vorteilhaft haben sich N,N-Dimethylacetamid, Methylenchlorid und Tetrahydrofuran erwiesen.

Als Mono-Alkalimetallsalze von Diolen der Formel (V) werden im erfindungsgemäßen Verfahren bevorzugt die Mono-Natrium-, Mono-Kalium- oder Mono-Cäsiumsalze eingesetzt. Insbesondere werden die Mono-Natriumsalze verwendet. R² steht bevorzugt für einen Rest der Formel (II), worin m eine ganze Zahl von 1-9, insbesondere gleich 1, 2 oder 3 ist, R⁶ Wasserstoff oder Methyl bedeutet und p gleich 1 oder 2 ist. Besonders bevorzugt steht der Rest R² für -[CH₂-CH₂]-, -[CH(CH₃)-CH₂]- oder -[CH₂-C(CH₃)₂-CH₂]-, wobei p gleich 1 ist.

R² steht ferner bevorzugt für einen Rest der Formel (III), bei dem r gleich 0 oder 1 ist. Besonders bevorzugt steht der Rest R² für

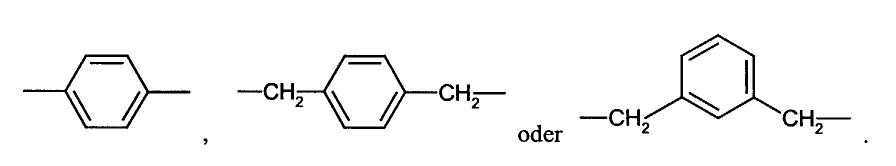

Bevorzugt wird als Mono-Alkalimetallsalz des Diols der Formel (V) Mono-Kaliumethylenglykolat und insbesondere Mono-Natrium-ethylenglykolat eingesetzt.

In einer bevorzugten Ausführungsform des erfindungsgemäßen Verfahrens wird das Mono-Alkalimetallsalz des Diols der Formel (V) in einem Gemisch mit einem Lösungsmittel eingesetzt.

Die Herstellung der Mono-Alkalimetallsalze von Diolen der Formel (V) ist dem Fachmann bekannt und erfolgt üblicherweise durch Umsetzung des Diols der Formel (V) mit einem Alkalimetallhydroxid, wobei das molare Verhältnis zwischen dem Diol und dem Alkalimetallhydroxid mindestens 2:1, bevorzugt (2-20):1, insbesondere (10-20):1 beträgt. Hierbei wird z.B. das überschüssige Diol der Formel (V) vorgelegt und das Alkalimetallhydroxid zudosiert. Auf diese Weise wird das Mono-Alkalimetallsalz des Diols im Gemisch mit dem Diol (V) als Lösungsmittel erhalten und kann unmittelbar in das erfindungsgemäße Verfahren eingesetzt werden.

Es hat sich ferner bewährt, bei der Herstellung der Mono-Alkalimetallsalze des Diols der Formel (V) neben dem überschüssigen Diol zusätzlich ein organisches Lösungsmittel B vorzulegen und anschließend das Alkalimetallhydroxid zuzugeben. Diese Verfahrensdurchführung ist insbesondere dann vorteilhaft, wenn das organische Lösungsmittel B zur azeotropen Trocknung des Reaktionsgemisches genutzt werden kann. Als organische Lösungsmittel B sind Ether, gegebenenfalls substituierte aliphatische Kohlenwasserstoffe, wie Nitroalkane oder Methylenchlorid, gegebenenfalls substituierte aromatische Kohlenwasserstoffe, wie Toluol, Nitroaromaten oder Chloraromaten, Amide und Ethylenglykol einsetzbar. Als besonders vorteilhaft haben sich N,N-Dimethylacetamid, Methylenchlorid und Tetrahydrofuran erwiesen. Eine destillative Entfernung des organischen Lösungsmittels B nach der Umsetzung kann ganz oder teilweise erfolgen. Im Fall der vollständigen Entfernung des organischen Lösungsmittels B wird das Mono-Alkalimetallsalz des Diols (V) wiederum im Gemisch mit dem überschüssigen Diol (V) erhalten. Bei nur anteiliger Entfernung des organischen Lösungsmittels B liegt das Mono-Alkalimetallsalz des Diols (V) entsprechend in einem Gemisch aus Diol (V) und organischem Lösungsmittel B vor und kann in dieser Form unmittelbar in das erfindungsgemäße Verfahren eingesetzt werden.

Für die Durchführung des erfindungsgemäßen Verfahrens ist es von Vorteil, wenn das Gemisch aus dem Mono-Alkalimetallsalz des Diols der Formel (V) und dem Diol der Formel (V) sowie gegebenenfalls dem organische Lösungsmittel B einen Wassergehalt von weniger als 5 %, vorzugsweise weniger als 2 % und besonders bevorzugt weniger als 0,2 % aufweist.

Der Begriff der simultanen Dosierweise im erfindungsgemäßen Verfahren umfasst auch, dass entweder das 1-Halogen-2,4-dinitrobenzol der Formel (IV) oder das Mono-Alkalimetallsalz des Diols der Formel (V) pro Zeiteinheit in einem Überschuss von maximal 20 mol-%, bevorzugt maximal 10 mol-% und insbesondere maximal 5 mol-%, bezogen auf den jeweils anderen Reaktionspartner, zudosiert werden kann. Hieraus folgt entsprechend, dass es möglich ist, dass bis zu 20 mol-%, vorzugsweise bis zu 10 mol-% und insbesondere bis zu 5 mol-% des Mono-Alkalimetallsalzes des Diols oder des 1-Halogen-2,4-dinitrobenzols im Reaktionsgefäß bereits vorgelegt werden.

Das molare Verhältnis des 1-Halogen-2,4-dinitrobenzols der Formel (IV) zum Mono-Alkalimetallsalz des Diols der Formel (V) beträgt 1:(1,05-2), vorzugsweise 1:1,1.

Im erfindungsgemäßen Verfahren wird üblicherweise bei Temperaturen von 20 bis 130°C, vorzugsweise 40 bis 80°C und besonders bevorzugt 60 bis 70°C gearbeitet. Als vorteilhaft hat es sich erwiesen, nach der Zudosierung unter Rühren eine Nachreaktion bei 20 bis 80°C über einen Zeitraum von bis zu 300 Minuten durchzuführen.

In einer weiteren bewährten Ausführungsform des erfindungsgemäßen Verfahrens werden das 1-Halogen-2,4-dinitrobenzol der Formel (IV) und das Mono-Alkalimetallsalz des Diols der Formel (V) in eine Vorlage eines organischen Lösungsmittels C dosiert. Besonders vorteilhaft ist es dabei, wenn das Mono-Alkalimetallsalz des Diols (V) im Gemisch mit dem Diol (V) und gegebenenfalls dem organischen Lösungsmittel B eingesetzt wird und das vorgelegte organische Lösungsmittel C identisch mit dem Diol (V) oder dem organischen Lösungsmittel B ist. Sofern das Mono-Alkalimetallsalz des Diols einen Rest R² der Formel (II) aufweist, so handelt es sich bei dem vorgelegten organischen Lösungsmittel C vorzugsweise um das zugehörige Diol der Formel (V) mit dem gleichen Rest R² der Formel (II). Prinzipiell sind als Lösungsmittel C aber auch Ether, gegebenenfalls substituierte aliphatische Kohlenwasserstoffe, wie Nitroalkane oder Methylenchlorid, gegebenenfalls substituierte aromatische Kohlenwasserstoffe, wie Toluol, Nitroaromaten oder Chloraromaten, Amide und Ethylenglykol geeignet. Als vorteilhaft haben sich N,N-Dimethylacetamid, Methylenchlorid und Tetrahydrofuran erwiesen.

Es hat sich ferner bewährt, die Reaktion unter Inertgas durchzuführen.

Die Isolierung des hergestellten 1-substituierten 2,4-Dinitrobenzols der Formel (I) erfolgt nach der Umsetzung der beiden Edukte üblicherweise durch Zugabe von Wasser zum Reaktionsgemisch und Abtrennung des ausgefallenen Produktes.

Das erfindungsgemäße Verfahren kann entweder in einem oder auch in zwei aufeinander folgenden Reaktoren durchgeführt werden. Im letzteren Fall dient der erste Reaktor hauptsächlich zur Eindosierung der beiden Edukte und der zweite Reaktor hauptsächlich der Zugabe von Wasser zwecks Ausfüllung des gewünschten 1-substituierten 2,4-Dinitrobenzols.

### Beispiel 1:

### Herstellung von Mono-Natrium-ethylenglykolat in Ethylenglykol

300 ml Toluol und 600 ml Ethylenglykol werden unter Stickstoff vorgelegt und anschließend 35,2 g (0,88 mol) Natriumhydroxid zugegeben. Die Mischung wird auf 102°C erhitzt und über einen Wasserabscheider Wasser entfernt. Nach fast vollständiger Entfernung des Wassers wird die gesamte Menge Toluol abdestilliert. Man erhält 586 g einer 12,6 %-igen Mono-Natrium-ethylenglykolat Lösung in Ethylenglykol mit einem Wassergehalt von ca. 0,1 %.

### Beispiel 2:

### Herstellung von 2-(2',4'-Dinitrophenoxy)-ethanol unter Verwendung der Mono-Natrium-ethylenglykolat Lösung in Ethylenglykol aus Beispiel 1

In einem 2 l Reaktionsgefäß mit Rührer werden 400 ml Ethylenglykol unter Stickstoff vorgelegt und auf 65°C erhitzt. Hierzu werden innerhalb von 5 h 162 g geschmolzenes 2,4-Dinitrochlorbenzol und 586 g der in Beispiel 1 erhaltenen 12,6 %-igen Mono-Natrium-ethylenglykolat Lösung in Ethylenglykol simultan zudosiert. Man lässt die Mischung bei 65°C eine Stunde nachrühren und gibt dann 600 ml Wasser hinzu. Der ausgefallene Niederschlag wird abfiltriert und mit 1000 ml Wasser nachgewaschen und getrocknet. Man erhält 165,3 g 2-(2',4'-Dinitrophenoxy)-ethanol in einer Reinheit von 99,2 %.

### Beispiel 3:

### Herstellung von Mono-Kalium-ethylenglykolat in Ethylenglykol

200 ml Toluol und 600 ml Ethylenglykol werden unter Stickstoff vorgelegt und anschließend 49,4 g Kaliumhydroxid zugegeben. Die Mischung wird auf 102°C erhitzt und über einen Wasserabscheider Wasser entfernt. Nach faktisch vollständiger Entfernung des Wassers wird die gesamte Menge Toluol abdestilliert. Man erhält 578 g einer 15,2 %-igen Mono-Kalium-ethylenglykolat Lösung in Ethylenglykol mit einem Wassergehalt von ca. 0,02 %.

### Beispiel 4:

### Herstellung von 2-(2',4'-Dinitrophenoxy)-ethanol unter Verwendung der Mono-Kalium-ethylenglykolat-Lösung aus Beispiel 3

In einem 2 l Reaktionsgefäß mit Rührer werden 200 ml Ethylenglykol und 40 ml der unter Beispiel 3 hergestellten Mono-Kalium-ethylenglykolat Lösung unter Stickstoff vorgelegt und auf 65°C erhitzt. Hierzu werden innerhalb von 5 h 162 g geschmolzenes 2,4-Dinitrochlorbenzol und die restliche Menge der Mono-Kalium-ethylenglykolat Lösung in Ethylenglykol simultan zudosiert. Man lässt die Mischung bei 65°C eine Stunde nachrühren und gibt dann 600 ml Wasser hinzu. Der ausgefallene Niederschlag wird abfiltriert und mit 1000 ml Wasser nachgewaschen und getrocknet. Man erhält 169,7 g 2-(2',4'-Dinitrophenoxy)-ethanol in einer Reinheit von 92 %.

### Beispiel 5:

### Herstellung von 2-(2',4'-Dinitrophenoxy)-ethanol unter Verwendung einer Lösung von 2,4-Dinitrochlorbenzol in N,N-Dimethylacetamid

In einem 2 l Reaktionsgefäß mit Rührer werden 200 ml Ethylenglykol und 40 ml einer 14,4 %-igen Mono-Natrium-ethylenglykolat Lösung unter Stickstoff vorgelegt und auf 65°C erhitzt. Hierzu werden innerhalb von 5 h 162 g geschmolzenes 2,4-Dinitrochlorbenzol und weitere 480 ml einer 14,4 %-igen Mono-Natrium-ethylenglykolat Lösung in Ethylenglykol simultan zudosiert. Man lässt die Mischung bei 65°C eine Stunde nachrühren und gibt dann 600 ml Wasser hinzu. Der ausgefallene Niederschlag wird abfiltriert und mit 1000 ml Wasser nachgewaschen und getrocknet. Man erhält 161,3 g 2-(2',4'-Dinitrophenoxy)-ethanol in einer Reinheit von 97 %.

## Patentansprüche

1. Verfahren zur Herstellung von 1-substituierten 2,4-Dinitrobenzolen der Formel (I) 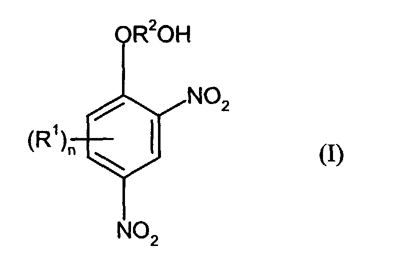
wobei R¹ unabhängig voneinander lineares oder verzweigtes C₁-C₂₀-Alkyl, C₆-C₁₈-Aryl, C₂-C₂₀-Acyl, COOH, COOR³, worin R³ für einen linearen oder verzweigten C₁-C₂₀-Alkylrest steht, SO₃H, SO₃R⁴, worin R⁴ für einen linearen oder verzweigten C₁-C₂₀-Alkylrest oder einen C₆-C₁₈-Arylrest, bei dem gegebenenfalls ein oder mehrere C-Atome durch O, S oder N ersetzt sind, steht, oder N(R⁵)₂, worin R⁵ unabhängig voneinander für Wasserstoff, einen linearen oder verzweigten C₁-C₂₀-Alkylrest oder einen C₂-C₂₀-Acylrest stehen, bedeuten, n eine ganze Zahl von 0-3 ist und
R² einen Rest der Formel (II) 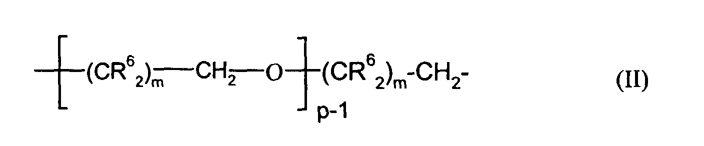
worin R⁶ unabhängig voneinander Wasserstoff oder einen C₁-C₅-Alkylrest bedeuten, m eine ganze Zahl von 1 bis 12 und p eine ganze Zahl von 1 bis 4 darstellt,
oder einen Rest der Formel (III) bedeutet, 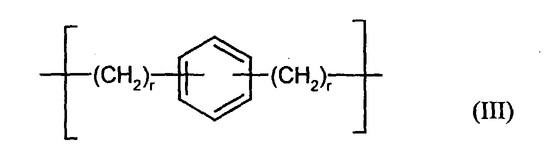
worin r unabhängig voneinander eine ganze Zahl von 0 bis 2 ist und gegebenenfalls ein oder mehrere Kohlenstoff-Atome des Phenylrings durch N, O oder S ersetzt sind,
durch Umsetzung von 1-Halogen-2,4-dinitrobenzolen der Formel (IV) 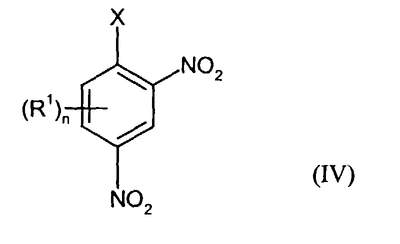
wobei X Halogen bedeutet und R^{¹} und n die für die Formel (I) genannten Bedeutungen besitzen,
mit Mono-Alkalimetallsalzen von Diolen der Formel (V)
HO-R²-OH (V)
wobei R² die für die Formel (I) genannten Bedeutungen besitzt,
welches dadurch gekennzeichnet ist, dass das 1-Halogen-2,4-dinitrobenzol der Formel (IV) und das Mono-Alkalimetallsalz des Diols der Formel (V) simultan dosiert und zur Reaktion gebracht werden.

2. Verfahren nach Anspruch 1, dadurch gekennzeichnet, dass in Formel (IV) X für Fluor, Chlor, Brom oder Iod steht.

3. Verfahren nach Anspruch 1 oder 2, dadurch gekennzeichnet, dass in Formel (IV) R¹ unabhängig voneinander lineares oder verzweigtes C₁-C₁₀-Alkyl, C₆-C₁₂-Aryl, C₂-C₆-Acyl oder N(R⁵)₂, worin R⁵ unabhängig voneinander für Wasserstoff, einen linearen oder verzweigten C₁-C₆-Alkylrest oder einen C₂-C₆-Acylrest stehen, bedeuten und unabhängig davon n gleich 0, 1 oder 2 ist.

4. Verfahren nach einem oder mehreren der Ansprüche 1 bis 3, dadurch gekennzeichnet, dass in der Formel (V) R² für einen Rest der Formel (II) steht, worin m eine ganze Zahl von 1 bis 9, insbesondere gleich 1, 2 oder 3 ist, R⁶ Wasserstoff oder Methyl bedeutet und p gleich 1 oder 2 ist.

5. Verfahren nach Anspruch 4, dadurch gekennzeichnet, dass der Rest R² für -[CH₂-CH₂]-, -[CH(CH₃)-CH₂]- oder -[CH₂-C(CH₃)₂-CH₂]- steht, wobei p gleich 1 ist.

6. Verfahren nach einem oder mehreren der Ansprüche 1 bis 5, dadurch gekennzeichnet, dass als Mono-Alkalimetallsalz des Diols der Formel (V) ein Mono-Natrium-, Mono-Kalium- oder Mono-Cäsiumsalz eingesetzt wird.

7. Verfahren nach Anspruch 6, dadurch gekennzeichnet, dass als Mono-Alkalimetallsalz des Diols der Formel (V) Mono-Kalium-ethylenglykolat und insbesondere Mono-Natrium-ethylenglykolat eingesetzt.

8. Verfahren nach einem oder mehreren der Ansprüche 1-7, dadurch gekennzeichnet, dass das Mono-Alkalimetallsalz des Diols der Formel (V) im Gemisch mit einem Lösungsmittel eingesetzt wird.

9. Verfahren nach Anspruch 8, dadurch gekennzeichnet, dass das Mono-Alkalimetallsalz des Diols der Formel (V) im Gemisch mit dem Diol der Formel (V) als Lösungsmittel und gegebenenfalls einem weiteren organischen Lösungsmittel, welches ausgewählt ist aus der Gruppe der Ether, gegebenenfalls substituierten aliphatischen Kohlenwasserstoffe, gegebenenfalls substituierten aromatischen Kohlenwasserstoffe und Amide, einen Wassergehalt von weniger als 5 %, bevorzugt von weniger als 2 % und besonders bevorzugt von weniger als 0,2 % aufweist.

10. Verfahren nach einem oder mehreren der Ansprüche 1 bis 9, dadurch gekennzeichnet, dass man die simultane Dosierung so durchführt, dass pro Zeiteinheit entweder das 1-Halogen-2,4-dinitrobenzol der Formel (IV) oder das Mono-Alkalimetallsalz des Diols der Formel (V) in einem Überschuss von bis zu 20 mol-%, bevorzugt bis zu 10 mol-% und insbesondere bis zu 5 mol-%, bezogen auf die jeweils andere Verbindung zudosiert wird.

11. Verfahren nach einem oder mehreren der Ansprüche 1 bis 10, dadurch gekennzeichnet, dass bis zu 20 mol-%, vorzugsweise bis zu 10 mol-%, besonders bevorzugt bis zu 5 mol-% des Mono-Alkalimetallsalzes des Diols der Formel (V) bereits vorgelegt werden.
